# EUROPEAN PATENT APPLICATION

(11) **EP 0 700 676 A1**
(43) Date of publication of application: **13.03.1996**
(21) Application number: 94202557.8
(22) Date of filing: 07.09.1994
(51) Int. Cl.: A61F 13/15, A61F 13/58

(54) **Disposable articles in a back to back configuration**

(71) Applicant: THE PROCTER & GAMBLE COMPANY, Cincinnati, Ohio 45202 (US)
(72) Inventor: Plumley, Julian Ashton, D-74589 Satteldorf (DE)
(74) Representative: Bottema, Johan Jan

(57) **Abstract**

The present invention relates to disposable articles, like for example absorbent sanitary napkins, having a garment facing side which is provided with an adhesive to temporarily adhere the article to the garment of a wearer. A release liner covers the adhesive on the garment facing side in order to protect it prior to use. The release liner has at least one article with its adhesive side joined to opposing sides of the release liner such that preferably one release liner is used for two articles.

## Description

### Field of the invention

The present invention relates to disposable articles, like for example absorbent sanitary napkins, having a garment facing side which is provided with an adhesive to temporarily adhere the article to the garment of a wearer. In particular the present invention relates to disposable absorbent articles having a release liner covering the adhesive on the garment facing side in order to protect it prior to use. The present invention is an improvement on the utilisation of the release liner for this purpose by having at least one article with its adhesive side joined to opposing sides of the release liner such that preferably one release liner is used for two articles.

### Background of the invention

In general the disposable absorbent articles according to the present invention have an adhesive on their garment facing side in order to adhere them to the garment of the user of such articles. This adhesive is adopted to maintain the article essentially fixed in relation to the garment and/or the wearer and thereby improve the utility of the particular article. In order to fulfil this objective the adhesive typically is protected prior to usage of the articles from soiling, premature adhesion to any particular surface and especially adhesion to itself which usually results in a state of the article which makes it unfit for use.

This protection prior to use of the adhesive on the garment facing side of an article is provided by a release liner. The release liner can be provided by any material desirable in this context, especially release liners of wax paper, paper which is coated with a release agent like for example silicon on the adhesive contacting side are well-known in the art, also release liners extending well beyond the periphery of the absorbent article in order to fold and wrap and enclose the absorbent article are well-know. These release liners are often made of polymeric film material like for example polyethylene film, polypropylene film and the like. Also release liners of woven or non-woven material have been reported to provide useful protection for the adhesive from premature soiling or wrongful adhesion to an undesired surface.

None of the references known to the applicant does, however, disclose a release liner which is utilised on both sides by having the respective adhesive side of an article attached so as to reduce the amount of release liner by up to 50 %.

It is therefore an objective of the present invention to provide disposable absorbent articles which have a reduction of up to 50 % of the release liner by joining the articles to both sides of the release liner.

It is in particular an objective of the present invention to provide sanitary napkins or panty liners having an adhesive on their garment facing side in a back to back pad construction where the sanitary napkins or panty liners are joined on both sides of a single release liner in registry of the products and preferably are packaged in groups of more than two sanitary napkins or two panty liners per package.

### Description of the invention

Many designs for disposable absorbent articles are known in the art. Disposable absorbent articles are used as sanitary napkins, catamenials, panty liners, adult incontinence products for light and medium incontinence or children's incontinence. Also contemplated by the present invention are disposable absorbent articles in particular those which can be used in pairs like for example disposable absorbent shoe inserts, disposable absorbent sweat pads or other disposable absorbent products. In addition the present invention may be applicable to other disposable products which are not inherently absorbent but cushion particular parts of the wearer's body like for example elbow pads or knee pads which can be applied by use of an adhesive to the garment of a wearer.

As mentioned above the present invention relates to a large variety of products having utility for absorbing liquids or cushioning body parts of wearers and which are applied to the desired location by means of a temporary fixation adhesive. The articles mainly contemplated by the applicant are, however, sanitary napkins, catamenials, panty liners and incontinence inserts. For all these disposable articles reference in the following will be made either to the generic term disposable articles or to sanitary napkins.

Disposable articles according to the present invention have a garment facing side and a garment facing adhesive on the garment facing side. The adhesive is provided in order to join the disposable article to the undergarment of a wearer so as to improve the utility of the article. The adhesive on the garment facing side of the article is protected by a release liner prior to use. The release liner has a first and a second surface which are both provided with the ability to easily delaminate from the adhesive on the articles. At least one disposable article each is placed on both the first and the second surface of the release liner and joined by means of the adhesive strength of the adhesive on the garment facing side to their respective side of the release liner.

Preferably two disposable articles are attached in particular in registry to the first and the second surface prior to use. The term "in registry" in this respect refers to disposable articles being placed on each side of the release liner such that their common periphery is essentially identical to their individual periphery.

The adhesive can be provided in any desired shape or form on the garment facing side of the disposable article or sanitary napkin. For example it can be applied as a longitudinal strip which extends the whole length of a sanitary napkin or it can be shorter than the whole length. Depending on the application process it can extend the full width perpendicular to the length of the article or leave the sides of the article free of adhesive. Also a multitude of adhesive strips or a random or designed web of adhesive can be supplied to the garment facing side of a sanitary napkin. It is also possible to have adhesive provided in a particular shape such as an endless strip following the periphery of the sanitary napkin with a short distance to that periphery or even directly adjacent that periphery while leaving the center of the sanitary napkin free of adhesive.

It is most desirable that the release liner be as small as possible while maintaining easy manufacturing capability of the sanitary napkin. In particular release liners being provided as endless bands having a width not exceeding the minimum dimension of the sanitary napkin perpendicular to its longitudinal axis have been found useful. Obviously the adhesive in this case is also applied in an area not exceeding the minimum dimension perpendicular to the longitudinal axis of the sanitary napkin. The sanitary napkins are placed onto this endless band of release liner in registry onto both surfaces of the release liner and then cut out at their longitudinal ends. Thereby a sandwich of sanitary napkin - release liner - sanitary napkin is formed.

The release liner ideally does not extend beyond the periphery of the sanitary napkins after it has been cut. For material consumption the adhesive and the release liner can even be shorter than the sanitary napkin. Typically the release liner under these conditions will be slightly larger in its width and/or length than the adhesive in order to allow a certain amount of placement variation when manufacturing the back to back sanitary napkins.

Since the release liner is shared by two sanitary napkins an individual packaging of these products is no longer possible. The option for double-packs of the articles is particularly interesting for such articles which are used in pairs. For sanitary napkins which are used individually double-packs may also be convenient e.g. to take them along when away from home. However, sanitary napkins or panty liners are typically stored and used always in the same place by an individual user. In this situation a bulk packaging of several back to back positioned sanitary napkins is attractive. This way of packaging allows to use the first of a pair of back-to-back sanitary napkins and return one sanitary napkin with the release liner remaining in place into the package e.g. into a cardboard box and use it later in the same fashion as known from typical prior art sanitary napkins.

A preferred process to make the disposable absorbent articles according to the present invention comprises the steps of providing a continuous thread of pairs of disposable absorbent articles. This continuous thread of pairs of disposable absorbent articles consists of a first and second continuous thread of disposable absorbent articles in a side by side relation such that the longitudinal ends of the articles are in registry with each other forming pairs. It is also possible to provide individually a first and a second continuous thread of disposable absorbent aricles which, however, requires careful alignment in order to obtain the desired side by side relation of the pairs of disposable absorbent articles when combining the threads later on.

Independent thereof a continuous thread of release liner having a first and a second release surface is provided. The next step is to apply the adhesive. The adhesive can either be applied to the garment facing side of the articles or to the release surfaces of the release liner or to combinations thereof. It is also not necessary that the adhesive for both articles of each pair be applied simultaneously.

A further step is to join the first and second thread of the absorbent articles with the surface of the release liner or vice versa. Again this can be done simultaneously or consecutive depending on the particular situation as will easily be appreciated by those skilled in the art.

The step of joining the articles to the release liner requires careful alignment of products (and adhesive if it is applied on the release liner). This is greatly simplified if a single thread of pairs of disposable absorbent articles is used since this thread only needs to be folded around the release liner ensuring that the articles are propperly aligned. Thereby the back-to-back sanitary napkin construction as discussed above in a continuous thread of articles and release liner is formed.

As a final step the articles and the release liner have to be severed into such back-to-back disposable absorbent article constructions as are desired. Particularly for pantiliners it has been found that a final die cut (or other full periphery cut out method) which creates the periphery of the pantiliners can be employed. If then the pantiliners are constructed with identical composition throughout their surface (no variation in laternal or longitudinal direction of any of the layers of the pantiliner) the alignment between pairs of pantiliners is automatically obtained upon the final cut out of the pantiliners.

Typically the adhesive is applied by coating (slot coating, curtain coating or spraying) in a continuous fashion as an endless line or strip. Alternatively the adhesive can be applied by a printing process again either as a continuous printing of a strip of adhesive or any desired configuration and contour of the adhesive within the periphery of the garment facing surface of the disposable absorbent article.

In the following the typical components of sanitary napkins are disclosed. However, many variations of the materials and the typical design of sanitary napkins are possible within the scope of the present invention which is defined by the claims and essentially relates to the utilisation of both sides of a release liner primarily to optimise of the material consumption.

Typically a sanitary napkin or panty liner comprises a topsheet forming the opposite side from the garment facing side and being liquid permeable. Further an absorbent core comprising one or more components is associated with the topsheet in liquid communication. Finally a backsheet forming the garment facing surface is also comprised in a sanitary napkin or panty liner and carries the panty fastening adhesive. These three components are described in more detail below.

### Topsheet

The topsheet is compliant, soft feeling, and non-irritating to the wearer's skin. The topsheet also can have elastic characteristics allowing it to be stretched in one or two directions. Further, the topsheet is fluid pervious permitting fluids (e.g., menses and/or urine) to readily penetrate through its thickness. A suitable topsheet can be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers.

Preferred topsheets for use in the present invention are selected from high loft nonwoven topsheets and apertured formed film topsheets. Apertured formed films are especially preferred for the topsheet because they are pervious to body exudates and yet non-absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. 5,006,394. Particularly preferred microapetured formed film topsheets are disclosed in U.S. patent 4,609,518 and U.S. patent 4,629,643. The preferred topsheet for the present invention comprises the formed film described in one or more of the above patents and marketed on sanitary napkins by The Procter & Gamble Company of Cincinnati, Ohio as "DRI-WEAVE."

Topsheets having not a homogeneous distribution of liquid passage ways but only a portion of the topsheet comprising liquid passage ways are also useful in the present invention. Typically such topsheets would have the liquid passage ways oriented such that they result in a centrally permeable and peripherally impermeable topsheet for liquids.

The body surface of the formed film topsheet can be hydrophilic so as to help liquid to transfer through the topsheet faster than if the body surface was not hydrophilic. In a preferred embodiment, surfactant is incorporated into the polymeric materials of the formed film topsheet such as is described in U.S. Patent Application Serial No. 07/794,745, "Absorbent Article Having A Nonwoven and Apertured Film Coversheet" filed on November 19, 1991. Alternatively, the body surface of the topsheet can be made hydrophilic by treating it with a surfactant such as is described in U.S. 4,950,254.

### Absorbent core

The absorbent core does not have to be a single entity. It can include the following components: (a) optionally a primary fluid distribution layer preferably together with a secondary fluid distribution layer; (b) a fluid storage layer; (c) optionally a fibrous ("dusting") layer underlying the storage layer; and (d) other optional components.

### a. Primary / Secondary Fluid Distribution Layer

One optional component of the absorbent cores according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized.

### b. Fluid Storage Layer

Positioned in fluid communication with, and typically underlying the primary or secondary distribution layers, is a fluid storage layer. The fluid storage layer can comprise absorbent gelling material, any usual absorbent material or combinations thereof. It preferably comprises absorbent gelling materials usually referred to as "hydrogels," "superabsorbent" "hydrocolloid" materials.

These absorbent gelling materials are capable of absorbing large quantities of aqueous body fluids, and are further capable of retaining such absorbed fluids under moderate pressures. These absorbent gelling materials can be in the form of discrete particles. The absorbent gelling materials can be dispersed homogeneously or non-homogeneously in a suitable carrier. Suitable carriers include cellulose fibers, in the form of fluff, tissue or paper, such as can conventionally be utilized in absorbent cores or in the form of tissues in single or multiple layers. Modified cellulose fibers such as the stiffened cellulose fibers can also be used.

Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., that lower rewet problems.

If dispersed non-homogeneously in a carrier, the storage layer can be locally homogeneous but have a distribution gradient in one or several directions within the dimensions of the storage layer. Non-homogeneous distribution can also refer to laminates of carriers enclosing absorbent gelling materials partially or fully. The laminate are preferred for pantiliners due to their thinness.

Suitable absorbent gelling materials for use herein will most often comprise a substantially water-insoluble, slightly crosslinked, partially neutralized, polymeric gelling material. This material forms a hydrogel upon contact with water. Such polymer materials can be prepared from polymerizable, unsaturated, acid-containing monomers. Suitable unsaturated acidic monomers for use in preparing the polymeric absorbent gelling material used in this invention include those listed in U.S. Patent 4,654,039 reissued as RE 32,649. Preferred monomers include acrylic acid, methacrylic acid, and 2-acrylamido-2-methyl propane sulfonic acid. Acrylic acid itself is especially preferred for preparation of the polymeric gelling material. Usual absorbent materials are those described as carrier above and can be in particular materials such as cellulose fibres, tissues or creped cellulose wadding.

### c. Optional Fibrous ("Dusting") Layer

An optional component for inclusion in the absorbent cores according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### e. Other Optional Components

The absorbent cores according to the present invention can include other optional components normally present in absorbent webs. For example, a reinforcing scrim can be positioned within the respective layers, or between the respective layers, of the absorbent cores. Such reinforcing scrims should be of such configuration as to not form interfacial barriers to fluid transfer, especially if positioned between the respective layers of the absorbent core. However, reinforcing scrims are usually not required for the absorbent structures according to the present invention.

Another component which can be included in the absorbent core according to the invention and preferably is provided close to or as part of the primary or secondary fluid distribution layer are odor control agents. Typically active carbon coated with or in addition to other odor control agents, in particular suitable zeolite or clay materials, are optionally incorporated in the absorbent core. These components can be incorporated in any desired form but often are included as discrete particles.

### Backsheet

The backsheet prevents the exudates absorbed and contained in the absorbent core from wetting articles that contact the sanitary napkin such as pants, pyjamas and undergarments. The backsheet (3) is impervious to liquids (e.g., menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet can comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm to about 0.051 mm.

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more cloth like appearance. Further, the backsheet can permit vapours to escape from the absorbent structure (i.e., be breathable) while still preventing exudates from passing through the backsheet.

### Panty Fastening Adhesive And Release-Liner

The backsheet typically forms the garment facing surface on which the panty fastening adhesive is placed.

Panty-fastening-adhesives can comprise any adhesive or glue used in the art for such purposes with pressure-sensitive adhesives being preferred. Suitable non-extensible adhesives are Century A-305-IV manufactured by the Century Adhesives Corporation, Instant Lock 34-2823 manufactured by the National Starch Company, 3 Sigma 3153 manufactured by 3 Sigma, and Fuller H-2238 ZP manufactured by the H.B. Fuller Co. Suitable adhesives are also described in U.S. Patent 4,917,697.

Suitable extensible adhesives for use as panty-fastening-adhesive include extensible adhesives, per se, and extensible adhesive/backsheet combinations. Any extensible adhesives known in the art can be used. Suitable extensible adhesive/backsheet combinations are for example non-extensible adhesive used on an extensible backsheet material such as 3 Sigma 2474 available from Anchor Continental, Inc., 3 Sigma Division, of Covington, Ohio; elastically stretchable adhesive films such as Findley adhesive 198-338, or an elastically stretchable adhesive film known as 3M XPO-0-014 available from the Minnesota Mining and Manufacturing Company of St. Paul, Minnesota; or spray adhesives such as 3M adhesive 1442 on a low modules elastic film. Other suitable panty-fastening-adhesives are shown in PCT International Patent Publication No. WO 92/04000; WO 93/01783 and WO 93/01785.

Sanitary napkins containing inextensible adhesives will typically only be capable of extension between the inextensible adhesive patches. Therefore, if inextensible adhesives are used, they are preferably applied in intermittent patterns such as for example intermittent dots, intermittent strips, random or designed filamentary patterns to permit the sanitary napkin to extend. If, on the other hand, the adhesive is extensible, the adhesive can be applied in continuous or intermittent patterns.

Prior to use of the absorbent article the panty fastening adhesive is protected from contamination and from sticking to any surface where this is not desired by a protective release liner such as a silicone coated release paper, a plastic film or any other easily removable cover. The protective release liner can be any of the well-known release liners in the art. In fact many of the commercially available release liners can simply be utilized by rendering the second surface releasable by the same method and in the same way as is usual with the first surface already.

Some release liners are already provided as double-face release liners due to the simplicity of the process for rendering them releasable. Suitable release liners are for example described in US Patent 4,917,697. Non-limiting examples of commercially available release liners suitable for being modified for the purposes of the present invention are for example BL30MG-A Silox E1/0 and BL30MG-A Silox 4P/O, both of which are manufactured by the Acrosil Corporation.

Other release liner materials are those known from individually wrapped sanitary napkins but which, without however, providing their wrapping function, can also perform the release liner function in a back-to-back pad construction. These materials are described in detail in US Patent 4,556,146 or PCT Application WO93/09743.

### Optional components of the absorbent articles

Optionally, the absorbent product of the present invention can comprise all those components which are known to be typical for the particularly intended product use. For example catamenials, panty liners and sanitary napkins often comprise components such as wings in order to improve their positioning and soiling protection performance. Leg elastication by one or several elastic strands is also common in the art of absorbent products. In general, all typically used components in absorbent products can also be comprised in the absorbent products according to the present invention.

## Claims

1. Disposable articles each having a garment facing side and a garment facing adhesive on said garment facing side, said adhesive being protected by a release liner prior to use, said release liner having a first and a second surface; said articles having a longitudinal axis and being characterised i n that at least one article is joined to said first surface of said release liner prior to use and at least one article is joined to said second surface of said release liner prior to use.

2. Disposable articles according to Claim 1 such that one article each is joined to said first and said second surface prior to use.

3. Disposable articles according to any of the preceding claims such that said articles are joined in registry to said first and second surface of said release liner prior to use.

4. Disposable articles according to any of the preceding claims such that said release liner has a surface area which is larger than the surface area of said adhesive.

5. Disposable articles according to any of the preceding claims such that said adhesive has an extension perpendicular to said longitudinal axis which is not exceeding the minimum extension of said articles perpendicular to said longitudinal axis.

6. Disposable articles according to any of the preceding claims such that said release liner does not extend beyond theperiphery of said articles.

7. Disposable articles according to any of the preceding claims such that said articles are absorbent articles.

8. Disposable absorbent articles according to claim 8 such that said articles are sanitary napkins or panty liners.

9. Disposable absorbent articles according to claims 8 or 9 such that said articles are packed in quantities of more than 2 articles per package.
